# EUROPÄISCHE PATENTSCHRIFT

(11) **EP 3 344 198 B1**
(45) Veröffentlichungstag und Bekanntmachung des Hinweises auf die Patenterteilung: **15.04.2026**
(21) Anmeldenummer: 16759739.2
(22) Anmeldetag: 30.08.2016
(51) Int. Cl.: A61F 5/01

(54) **RASTGELENK**
LOCKING HINGE
ARTICULATION À ENCLIQUETAGE

(30) Priorität: 31.08.2015 DE 102015114540
(43) Veröffentlichungstag der Anmeldung: 11.07.2018
(73) Patentinhaber: Ottobock SE & Co. KGaA, 37115 Duderstadt (DE)
(72) Erfinder: SCHILLING, Matthias, 37345 Weißenborn-Lüderode (DE); KROLL-ORYWAHL, Olaf, 37154 Northeim (DE)
(74) Vertreter: Gramm, Lins & Partner Patent- und Rechtsanwälte PartGmbB
(86) Internationale Anmeldenummer: PCT/EP2016/070394
(87) Internationale Veröffentlichungsnummer: WO 2017/037049

(56) Entgegenhaltungen:
- EP-A1- 1 579 829
- EP-A2- 1 457 174
- CN-U- 201 745 635
- CN-U- 203 623 762
- DE-A1- 102007 062 961
- US-A- 4 711 242

## Beschreibung

Die Erfindung betrifft ein Rastgelenk mit einem ersten Bauteil und einem zweiten Bauteil, die um eine Schwenkachse relativ zueinander verschwenkbar aneinander gelagert sind, wobei das erste Bauteil eine Rastausnehmung und das zweite Bauteil ein Rastelement aufweist, das eingerichtet ist, in die Rastausnehmung einzudringen und so eine Schwenkbewegung des ersten Bauteils relativ zu dem zweiten Bauteil in wenigstens eine Richtung zu verhindern, sobald das erste Bauteil relativ zu dem zweiten Bauteil eine Rastposition erreicht. Die Erfindung betrifft zudem eine Orthese, insbesondere eine Knieorthese, mit einem derartigen Rastgelenk.

Aus der CN 203623762 U ist ein solches Rastgelenk mit den Merkmalen des Oberbegriffes des Anspruchs 1 bekannt.

Derartige Rastgelenke werden insbesondere in der Orthopädietechnik als Gelenke für Orthesen eingesetzt, können jedoch auch in vielen anderen Anwendungen verwendet werden. Wird das Rastgelenk beispielsweise in einer Knieorthese eingesetzt, soll das Gelenk beispielsweise in der Schwungphase eines Schrittes die Extension, also die Streckung des Kniegelenkes, zulassen. Sobald das Knie die volle Extension erreicht hat, also beispielsweise gestreckt ist, dringt das Rastelement in die Rastausnehmung ein, da die Rastposition der beiden Bauteile zueinander erreicht ist. Dadurch wird erreicht, dass beim Aufsetzen des Fußes eine Verschwenkung der beiden Bauteile relativ zueinander in Beugerichtung blockiert ist, sodass eine Belastung des Rastgelenkes möglich ist.

Aufgrund verschiedener Ursachen ist es jedoch möglich, dass beispielsweise das Knie in einer Schwungphase die geforderte Extension nicht erreicht, da beispielsweise der Patient motorisch eingeschränkt ist oder beispielsweise stolpert. In diesem Fall kann das Rastelement nicht in die Rastausnehmung eindringen, sodass eine Bewegung in Beugerichtung des Gelenkes nicht blockiert werden kann. Wird das Bein und damit das Rastgelenk in der Folge belastet, ist eine Verschwenkung der beiden Bauteile in Beugerichtung relativ zueinander weiterhin möglich, die vom Patienten erwartete Unterstützung durch das Gelenk bleibt aus und es droht der Sturz.

Aus der DE 20 2006 007 451 U1 ist es daher bekannt, zwischen den beiden Bauteilen ein Funktionselement anzuordnen, das über zumindest einen Teil seines Umfangs eine Verzahnung aufweist, in die das Rastelement eingreift. Bei einem Verschwenken der beiden Bauteile relativ zueinander beispielsweise bei der Extension oder Streckung eines Kniegelenkes wird daher diese Schwenkbewegung nicht behindert, während eine Bewegung in die entgegengesetzte Richtung durch das jeweilige Eingreifen des Rastelementes in die Verzahnung an der Außenseite des Funktionselementes unterbunden wird. Nachteilig ist jedoch, dass das Rastelement bei jedem Schritt und bei jeder Bewegung des Gelenkes über die einzelnen Zähne der Verzahnung hinweggleitet, sodass diese einer relativ starken Abnutzung ausgesetzt sind. Zudem entstehen bei jedem Schritt Geräusche, wenn das Rastelement über die einzelnen Zähne der Verzahnung hinweggleitet und in die Zwischenräume zwischen benachbarten Zähnen eingreift und hineinfällt. Diese können als unangenehm und störend empfunden werden.

Der Erfindung liegt daher die Aufgabe zugrunde, ein Rastelement so weiterzuentwickeln, dass diese Nachteile behoben werden.

Die Erfindung löst die gestellte Aufgabe durch ein Rastgelenk gemäß dem Oberbegriff des Anspruchs 1, dass sich dadurch auszeichnet, dass das Rastelement eingerichtet ist, in wenigstens zwei verschiedenen Rastpositionen unterschiedlich tief in die Rastausnehmung einzudringen, und so in beiden Rastpositionen die Schwenkbewegung des ersten Bauteils relativ zu dem zweiten Bauteil in der einen Richtung zu verhindern, wobei das Rastelement und/oder die Rastausnehmung an wenigstens einer Seitenfläche wenigstens eine Stufe aufweist.

Auf diese Weise wird erreicht, dass mehrere Rastpositionen vorhanden sind, sodass selbst für den Fall, dass beispielsweise beim Verschwenken des Gelenkes die finale und endgültig gewünschte Position, beispielsweise der Extensionsanschlag, nicht erreicht wird, eine Voreinrastung in eine vor diesem Anschlag liegende Rastposition erfolgen kann. Werden also das erste Bauteil und das zweite Bauteil um die gemeinsame Schwenkachse herum relativ zueinander verschwenkt, was beispielsweise bei der bereits beschriebenen Extension eines Kniegelenkes erfolgen kann, erreichen die beiden Bauteile während dieser Bewegung eine erste der verschiedenen Rastpositionen. Zu diesem Zeitpunkt dringt das Rastelement in die Rastausnehmung ein, sodass ab diesem Zeitpunkt eine Schwenkbewegung der beiden Bauteile in die entgegengesetzte Richtung nicht mehr möglich ist. Bei dem bereits beschriebenen Beispiel einer Knieorthese wird diese erste Rastposition vorzugsweise erreicht, bevor die endgültige Rastposition, insbesondere der bereits beschriebene Extensionsanschlag, erreicht wird. Damit entsteht eine Sicherung, die insbesondere bei der Verwendung des Rastgelenkes in orthopädietechnischen Einrichtungen, wie beispielsweise Orthesen und insbesondere Knieorthesen, von Vorteil ist.

Werden die beiden Bauteile in die ursprüngliche Richtung weiter relativ zu-einander verschwenkt, wird also beispielsweise das Rastgelenk der Knieorthese weiter in Extensionsrichtung bewegt, erreichen die beiden Bauteile zu einem späteren Zeitpunkt eine zweite der verschiedenen Rastpositionen. Zu diesem Zeitpunkt dringt das Rastelement tiefer in die Rastausnehmung ein, sodass ab diesem Moment auch eine Schwenkbewegung in die entgegengesetzte Richtung bis zur ersten erreichten Rastposition nicht mehr möglich ist. Diese zweite Rastposition kann die endgültig gewünschte Rastposition, beispielsweise der Extensionsanschlag, sein. Alternativ dazu können natürlich auch mehr als zwei unterschiedliche Rastpositionen erreicht werden. Da dabei nicht, wie aus dem Stand der Technik, ein Rastelement über eine Zahnreihe hinweggleitet, kommt es zu einem deutlich geringeren Verschleiß und zu nahezu keiner Geräuschentwicklung.

Vorteilhafterweise bewegt sich das Rastelement zum Eindringen in die Rastausnehmung in radialer Richtung bezüglich der Schwenkachse. Besonders vorteilhafterweise bewegt es sich nach radial innen, um in die Rastausnehmung eindringen zu können. Erfindungsgemäß weist das Rastelement und / oder die Rastausnehmung an wenigstens einer Seitenfläche wenigstens eine Stufe auf. Verfügt das Rastelement beispielsweise über eine Stufe, wird es bei Erreichen der ersten Rastposition zwischen dem ersten Bauteil und dem zweiten Bauteil bis zu dieser Stufe in die Rastausnehmung eindringen. Ein weiteres Eindringen ist jedoch erst dann möglich, wenn die zweite Rastposition erreicht wird, und das Rastelement weiter in die Rastausnehmung eindringen kann. Analog verhält es sich, wenn die Rastausnehmung an einer ihrer Seitenflächen eine entsprechende Stufe aufweist. Natürlich können auch sowohl das Rastelement als auch die Rastausnehmung wenigstens eine Stufe aufweisen. Je nach gewünschter Anzahl von unterschiedlichen Rastpositionen können das Rastelement und / oder die Rastausnehmung auch mehrere Stufen aufweisen. Vorzugsweise weist die wenigstens eine Stufe des Rastelementes eine andere Breite und / oder eine andere Tiefe auf, als die wenigstens eine Stufe der Rastausnehmung. Natürlich können auch die Stufen des Rastelementes und / oder die Stufen der Rastausnehmung untereinander unterschiedliche Breiten und / oder Tiefen aufweisen. Auf diese Weise sind durch relativ einfache Fertigungsschritte eine große Anzahl ggf. unterschiedlich weit voneinander beabstandeter Rastpositionen erreichbar. Natürlich können auch äquidistant angeordnete Rastpositionen auf diese Weise erreicht werden.

In einer bevorzugten Ausführungsform verfügt das Rastelement und / oder die Rastausnehmung über wenigstens eine relativ zur Radialrichtung abgeschrägte Seitenfläche. Als besonders vorteilhaft hat sich herausgestellt, wenn sowohl das Rastelement als auch die Rastausnehmung jeweils eine abgeschrägte Seitenfläche aufweisen, wobei die beiden abgeschrägten Seitenflächen den gleichen Winkel relativ zur Radialrichtung aufweisen. Auf diese Weise dringt das Rastelement bei Überschreiten der ersten Rastposition kontinuierlich weiter in die Rastausnehmung ein, wobei die beiden abgeschrägten Seitenflächen des Rastelementes und der Rastausnehmung aneinander abgleiten. Bis das Rastelement vollständig, also soweit es durch die konstruktive Ausgestaltung möglich ist, in die Rastausnehmung eingedrungen ist, ist folglich jede Position zwischen dem ersten Bauteil und dem zweiten Bauteil eine Rastposition, in der das Rastgelenk eine Schwenkbewegung in die umgekehrte Richtung verhindert.

In einer bevorzugten Ausgestaltung der Erfindung liegt zwischen den beiden am weitesten voneinander beabstandeten Rastpositionen ein Schwenkwinkel um die Schwenkachse zwischen 0° und 30°, bevorzugt zwischen 5° und 15°, besonders bevorzugt von 10°. Natürlich sind jedoch auch andere Winkelbereiche je nach gewünschter Anwendung möglich.

Es hat sich als vorteilhaft herausgestellt, wenn das Rastelement derart angeordnet ist, dass es beim Verschwenken des ersten Bauteiles relativ zu dem zweiten Bauteil auf einer Fläche des ersten Bauteils entlanggleitet, in der sich die Rastausnehmung befindet. Als besonders vorteilhaft hat sich herausgestellt, wenn das Rastelement in diesem Fall federbelastet in Richtung auf die Fläche des ersten Bauteiles und bei Erreichen einer Rastposition auch in Richtung auf die Rastausnehmung ist. Auf diese Weise wird sichergestellt, dass das Rastelement sicher in die Rastausnehmung hineingleitet und so eine Bewegung in die entgegengesetzte Schwenkrichtung sicher verhindert.

Zu einem späteren Zeitpunkt kann es durchaus gewünscht sein, dass das Rastgelenk wieder in beide Richtungen bewegbar und somit das erste Bauteil relativ zu dem zweiten Bauteil in beide Richtungen um die Schwenkachse verschwenkbar ist. Dafür muss das Rastelement aus der Rastausnehmung entfernt werden. Daher ist es von Vorteil, wenn das Rastgelenk eine Löseeinrichtung aufweist, die eingerichtet, das Rastelement aus der Rastausnehmung zu entfernen. Diese kann auf unterschiedlichste Weise ausgebildet sein. Denkbar ist beispielsweise eine mechanische oder magnetische Löseeinrichtung. Insbesondere bei einer magnetisch ausgebildeten Löseeinrichtung kann ein Elektromagnet vorhanden sein, der mit seiner durch den elektrischen Strom schaltbaren magnetischen Anziehungskraft das Rastelement aus der Rastausnehmung herausziehen kann. Dadurch wird eine ggf. vorhandene Federkraft, die das Rastelement in die Rastausnehmung hineindrückt, überwunden, sodass in diesem Fall das erste Bauteil relativ zu dem zweiten Bauteil frei schwenkbar ist. Die maximal mögliche Schwenkbewegung wird dabei allenfalls durch konstruktive Anschläge oder sonstige Ausgestaltungen beeinträchtigt.

Eine mechanische Löseeinrichtung kann beispielsweise über einen Hebel, einen Seilzug oder eine Feder betätigt werden. Zudem sind pneumatische und/oder hydraulische Löseeinrichtungen denkbar. Durch alle genannten Löseeinrichtungen oder andere denkbare Ausgestaltungen einer Löseeinrichtung wird das Rastelement aus der Rastausnehmung herausgezogen und somit die Schwenkbewegung der beiden Bauteile relativ zueinander wieder in beide Richtungen ermöglicht.

Die Erfindung löst die gestellte Aufgabe zudem durch eine orthopädietechnische Einrichtung, die wenigstens ein Rastgelenk der hier beschriebenen Art aufweist. Vorteilhafterweise werden zwei Rastgelenke verwendet, die auf unterschiedlichen Seiten, beispielsweise medial und lateral, eines Körperteils, an dem die Orthese anzulegen ist, angeordnet.

Eine orthopädietechnische Einrichtung kann beispielsweise eine Orthese oder eine Prothese sein. Eine derartige Orthese oder Prothese kann für einen Arm, ein Bein, einen Fuß oder eine Hand verwendet werden, sodass das hier beschriebene Rastgelenk oder die Rastgelenke für einen Ellenbogen, eine Schulter, ein Knie, eine Hüfte, einen Knöchel oder ein Handgelenk verwendet werden kann.

Mit Hilfe einer Zeichnung wird nachfolgend ein Ausführungsbeispiel der vorliegenden Erfindung näher erläutert. Es zeigen:
- Figuren 1a bis 1c -: die schematische Darstellung eines Rastgelenkes in einer Teilschnittansicht und
- Figuren 2 bis 7d-: unterschiedliche Ausgestaltungen einer Rastausnehmung und eines Rastelementes.

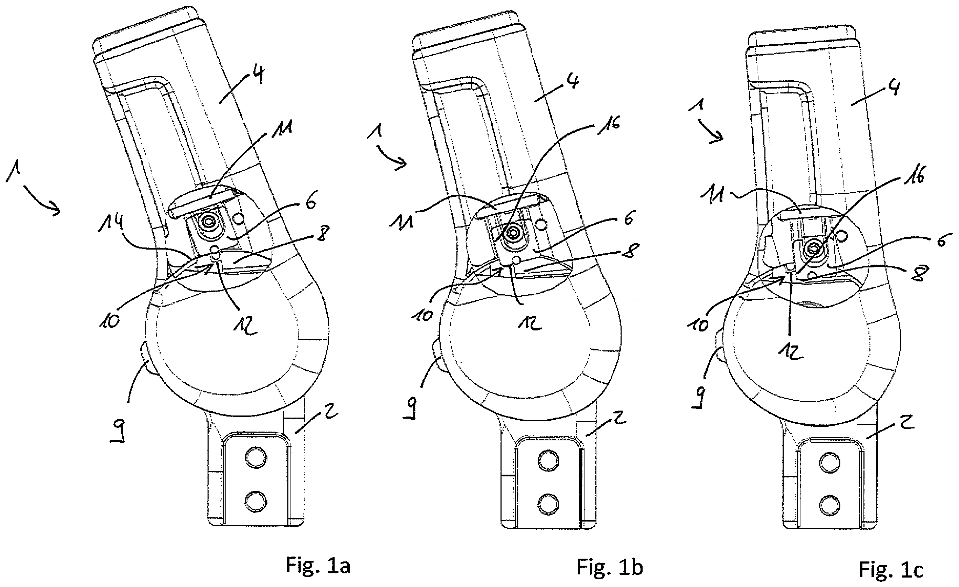

In den Figuren 1a bis 1c ist ein Rastgelenk 1 dargestellt. Es verfügt über ein erstes Bauteil 2 und ein zweites Bauteil 4. An dem zweiten Bauteil 4 befindet sich ein Rastelement 6, während das erste Bauteil 2 über eine Rastausnehmung 8 verfügt. In den Figuren 1a bis 1c sind das erste Bauteil 2 und das zweite Bauteil 4 in drei unterschiedlichen Positionen relativ zueinander dargestellt.

Die Rastausnehmung 8 verfügt in den Figuren 1a bis 1c über eine erste Seitenwand 10, die in den Figuren 1a bis 1c eine Stufe 12 aufweist.

In der in Figur 1a gezeigten Situation liegt das Rastelement 6 an einer Fläche 14 an, die Teil des ersten Bauteils 2 ist. Wird das zweite Bauteil 4 um eine in den Figuren 1a bis 1c nicht dargestellte Schwenkachse verschwenkt, gleitet das Rastelement 6 auf dieser Fläche 14 ab.

In Figur 1b haben das erste Bauteil 2 und das zweite Bauteil 4 die erste Rastposition erreicht. Man erkennt, dass das Rastelement 6 bis zur Stufe 12 in die Rastausnehmung 8 eingedrungen ist. Eine weitere Verschwenkung des zweiten Bauteils 4 relativ zum ersten Bauteil 2 ist nur im Uhrzeigersinn, nicht jedoch in die entgegengesetzte Richtung möglich, da das Rastelement 6 mit seiner zweiten Seitenwand 16 an der Stufe 12 der ersten Seitenwand 10 anliegt.

Figur 1c zeigt die Situation nach Erreichen der zweiten Rastposition zwischen dem ersten Bauteil 2 und dem zweiten Bauteil 4. Das Rastelement 6 ist vollständig in die Rastausnehmung 8 eingedrungen und liegt nun mit seiner zweiten Seitenwand 16 am unteren Teil der ersten Seitenwand 10 an.

Soll das in den Figuren 1a bis 1c gezeigte Rastelement 1 wieder so bewegt werden, dass das zweite Bauteil 4 relativ zum ersten Bauteil 2 entgegen dem Uhrzeigersinn verdreht und verschwenkt werden kann, muss das Rastelement 6 aus der Rastausnehmung 8 entfernt werden.

In den Figuren 1a bis 1c ist zudem eine Löseeinrichtung 11 dargestellt, durch die das Rastelement 6 aus der in Figur 1c gezeigten Position in der Rastausnehmung 8 wieder entfernbar ist. Im gezeigten Ausführungsbeispiel ist die Löseeinrichtung 11 als elektromagnetische Löseeinrichtung 11 ausgeführt, sodass durch die Beaufschlagung mit einem Strom ein magnetisches Feld erzeugt werden kann, durch das das Rastelement 6 aus der in Figur 1c gezeigten Position entfernt werden kann.

Zudem ist eine Notentriegelung 9 dargestellt, durch die das Rastgelenk jederzeit mechanisch entriegelt werden kann, sofern dies notwendig erscheint.

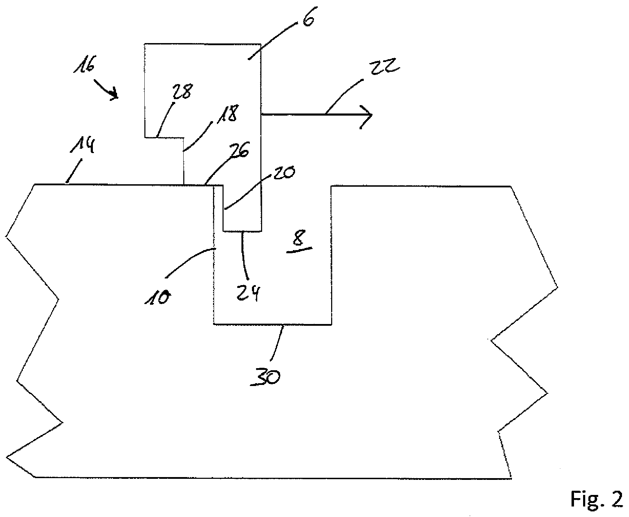

Figur 2 zeigt die schematische Darstellung eines Teils eines Rastelementes 6 und einer Rastausnehmung 8. Man erkennt, dass die erste Seitenwand 10 keinerlei Strukturen aufweist, sondern sich in radialer Richtung auf die Schwenkachse zu erstreckt. Die Schwenkachse ist in Figur 2 nicht dargestellt. Die zweiten Seitenwand 16 des Rastelementes 6 verfügt hingegen über eine erste Stufe 18 und eine zweite Stufe 20. Wird nun das erste Bauteil 2 relativ zum zweiten Bauteil 4 so verschwenkt, dass das Rastelement 6 in Richtung des Pfeiles 22 bewegt wird, wird zunächst eine Unterseite 24 des Rastelementes 6 auf der Fläche 14 angeordnet sein. In einer bestimmten Position des ersten Bauteils 2 relativ zum zweiten Bauteil 4 wird die erste Rastposition erreicht, sodass das Rastelement bis zur ersten Stufenfläche 26 in die Rastausnehmung 8 eindringen kann. Diese Situation ist in Figur 2 dargestellt. Wird das Rastelement 6 weiter entlang des Pfeiles 22 verschoben, wird zu einem späteren Zeitpunkt eine zweite Rastposition erreicht, in der auch die erste Stufe 18 in die Rastausnehmung 8 eindringen kann, wobei das Rastelement 6 soweit in die Rastausnehmung 8 eindringt, bis die zweite Stufenfläche 28 auf der Fläche 14 anliegt. Erst zu einem noch späteren Zeitpunkt wird eine dritte Rastposition erreicht, in der das Rastelement 6 vollständig in die Rastausnehmung 8 eindringen kann, bis die Unterseite 24 an einem Ausnehmungsgrund 30 der Rastausnehmung 8 anliegt.

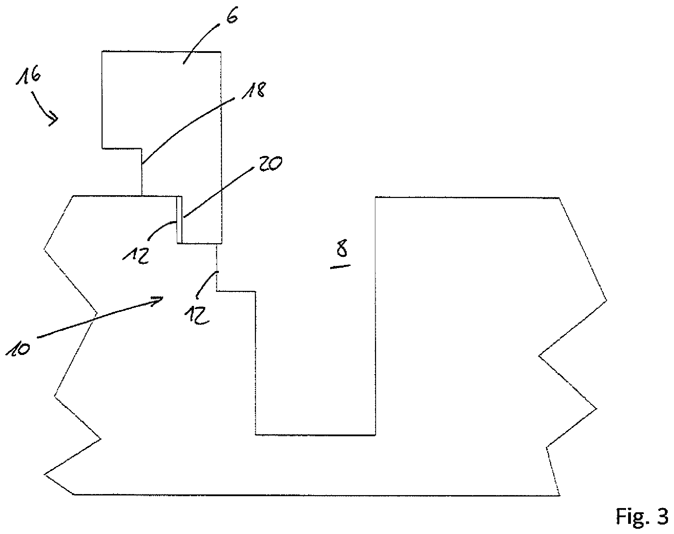

In Figur 3 weist die zweite Seitenwand 16 des Rastelementes 6 ebenfalls die erste Stufe 18 und die zweite Stufe 20 auf. Anders als in der in Figur 2 gezeigten Ausgestaltung verfügt nun jedoch auch die erste Seitenwand 10 der Rastausnehmung 8 über zwei Stufen 12, die im in Figur 3 gezeigten Ausführungsbeispiel die gleiche Höhe und Breite wie die erste Stufe 18 und die zweite Stufe 20 des Rastelementes 6 aufweisen. Während die in Figur 2 gezeigte Ausführungsform von Rastelement 6 und Rastausnehmung 8 zu drei voneinander äquidistant beabstandeten Rastpositionen führt, verfügt die in Figur 3 gezeigte Ausführungsform über fünf Rastpositionen, bei denen jeweils das Rastelement 6 weiter in die Rastausnehmung 8 eindringt.

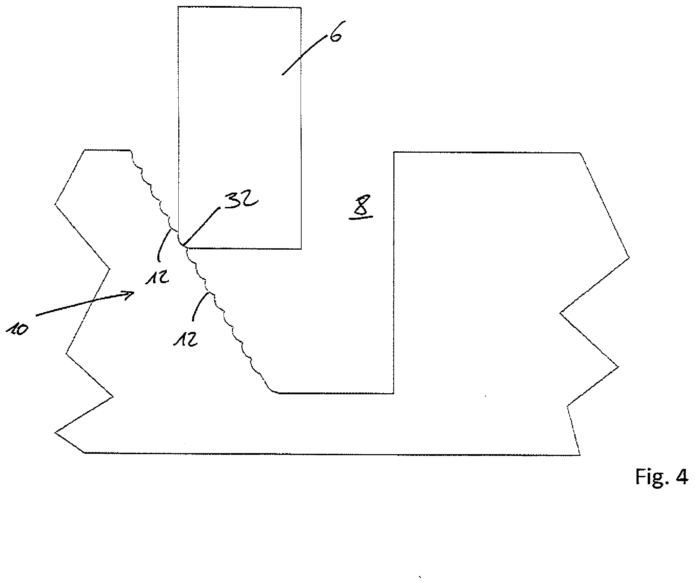

Figur 4 zeigen eine weitere Ausgestaltung eines Rastelementes 6 und einer Rastausnehmung 8. Die erste Seitenwand 10 der Rastausnehmung 8 verfügt über eine Mehrzahl von abgerundeten Stufen 12, deren Kontur der Kontur einer abgerundeten Ecke 32 des Rastelementes 6 entsprechen. Wird das Rastelement 6 in Figur 4 nach rechts bewegt, wird immer dann, wenn die abgerundete Ecke 32 in die jeweils nächste Stufe 12 eindringt auf das Rastelement 6 weiter in die Rastausnehmung 8 eindringen, sodass immer zu diesen Zeitpunkten die jeweils nächste Rastposition erreicht wird.

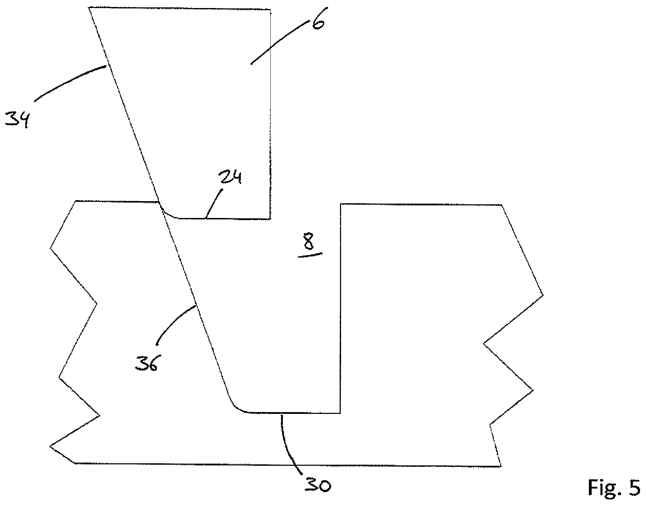

In Figur 5 ist die erste Seitenwand 10 eines nicht erfindungsgemäßen Rastelementes 6 als abgeschrägte Seitenfläche 34 ausgebildet. Auch die zweite Seitenwand 16 der Rastausnehmung 8 ist als abgeschrägte Seitenfläche 36 ausgebildet, wobei gut zu erkennen ist, dass die abgeschrägten Seitenflächen 34, 36 den gleichen Winkel relativ zur Radialrichtung, die sich in Figur 5 nach unter erstreckt, aufweisen. Daher können die beiden abgeschrägten Seitenflächen 34, 36 aneinander abgleiten, wenn das Rastelement 6 in Figur 5 nach rechts bewegt wird. Die verschiedenen Rastpositionen sind somit kontinuierlich verteilt bis das Rastelement 6 vollständig in der Rastausnehmung 8 aufgenommen ist und die Unterseite 24 am Ausnehmungsgrund 30 anliegt.

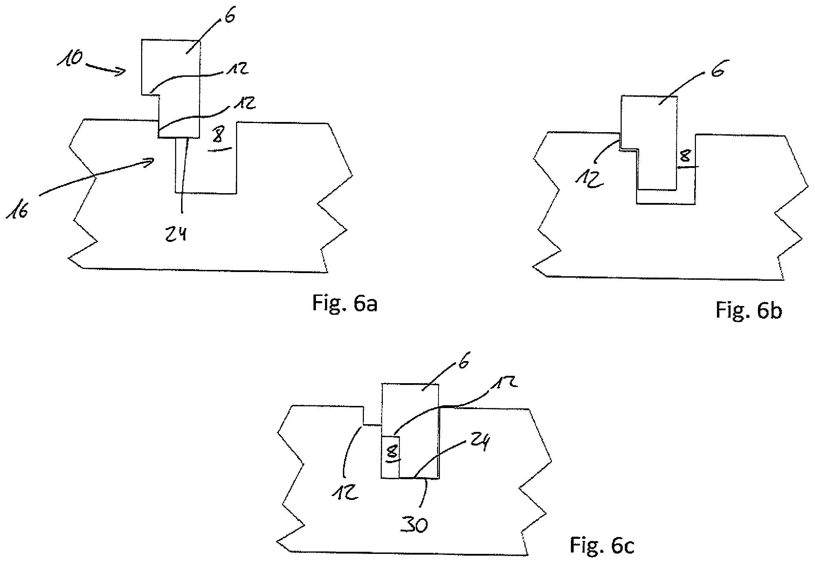

In den Figuren 6a bis 6c sind verschiedene Positionen des Rastelementes 6 und der Rastausnehmung 8 gezeigt. Sowohl die erste Seitenwand 10 als auch die zweiten Seitenwand 16 verfügen jeweils über eine Stufe 12, die in unterschiedlichen Positionen aneinander anliegen können. In Figur 6a ist diese Situation bei Erreichen der ersten Rastposition dargestellt. Die Unterseite 24 des Rastelementes 6 liegt auf der Stufe 12 der zweiten Seitenwand 16 an. In Figur 6b ist das Rastelement 6 relativ zur Rastausnehmung 8 nach rechts verschoben worden, sodass hier die beiden Stufen 12 aneinander anliegen. In Figur 6c ist die dritte und letzte Rastposition erreicht, indem das Rastelement 6 relativ zur Rastausnehmung 8 weiter nach rechts verschoben wurde. Hier liegt die Unterseite 24 am Ausnehmungsgrund 30 an.

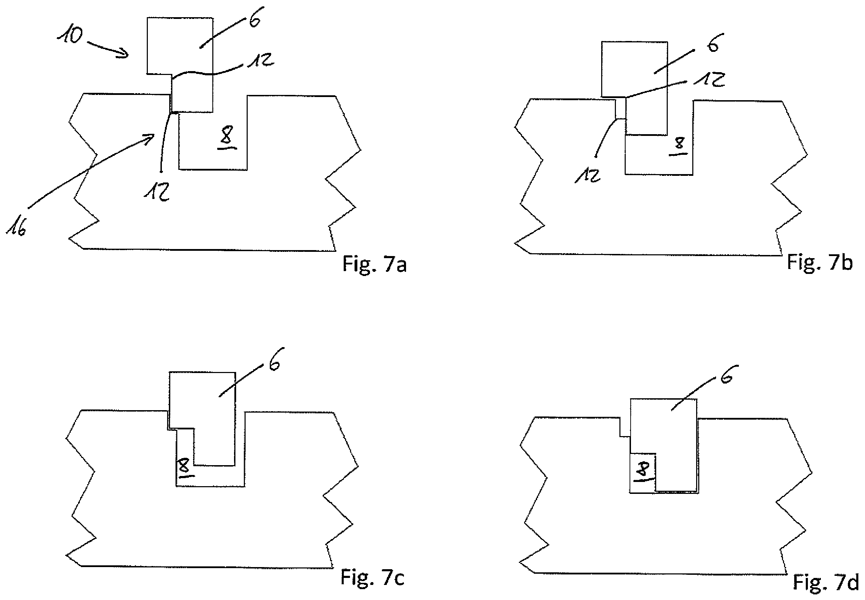

In den Figuren 7a bis 7d ist eine weitere Ausführungsform des Rastelementes 6 sowie der Rastausnehmung 8 dargestellt.

Man erkennt, dass sowohl die erste Seitenwand 10 des Rastelementes 6 als auch die zweite Seitenwand 16 der Rastausnehmung 8 über jeweils eine Stufe 12 verfügen, die jedoch im gezeigten Ausführungsbeispiel unterschiedlich breit sind. In den Figuren 7a bis 7d wird das Rastelement 6 relativ zur Rastausnehmung 8 immer weiter nach rechts verschoben, sodass es immer tiefer in die Rastausnehmung 8 eindringen kann. Durch die unterschiedliche Breite der Stufen, die zudem eine unterschiedliche Höhe aufweisen, werden die in den Figuren 7a bis 7d gezeigten vier Rastpositionen ermöglicht, wobei das Rastelement 6 mit fortschreitender Verschiebung nach rechts relativ zur Rastausnehmung 8 immer tiefer in die Rastausnehmung 8 eindringt.

Diese Ausgestaltung hat den Vorteil, dass auf besonders einfach herstellbare Weise eine relativ große Anzahl verschiedener Rastpositionen erreicht und realisiert werden kann, sodass der Fertigungsaufwand gering und die erreichte Zusatzsicherheit für das Rastgelenk hoch sind.

### Bezugszeichenliste

- 1: Rastgelenk
- 2: erstes Bauteil
- 4: zweites Bauteil
- 6: Rastelement
- 8: Rastausnehmung
- 9: Notentriegelung
- 10: erste Seitenwand
- 11: Löseeinrichtung
- 12: Stufe
- 14: Fläche
- 16: zweite Seitenwand
- 18: erste Stufe
- 20: zweite Stufe
- 22: Pfeil
- 24: Unterseite
- 26: erste Stufenfläche
- 28: zweite Stufenfläche
- 30: Ausnehmungsgrund
- 32: abgerundete Ecke
- 34: abgeschrägte Seitenfläche
- 36: abgeschrägte Seitenfläche

## Patentansprüche

1. Rastgelenk (1) mit
einem ersten Bauteil (2) und
einem zweiten Bauteil (4)
die um eine Schwenkachse relativ zueinander schwenkbar aneinander gelagert sind,
wobei das erste Bauteil (2) eine Rastausnehmung (8) und das zweite Bauteil (4) ein Rastelement (6) aufweist, das eingerichtet ist, in die Rastausnehmung (8) einzudringen und so eine Schwenkbewegung des ersten Bauteils (2) relativ zu dem zweiten Bauteil (4) in wenigstens einer Richtung zu verhindern, sobald das erste Bauteil (2) relativ zu dem zweiten Bauteil (4) eine Rastposition erreicht,
**dadurch gekennzeichnet, dass** das Rastelement (6), eingerichtet ist, in wenigstens zwei verschiedenen Rastpositionen unterschiedlich tief in die Rastausnehmung (8) einzudringen und so in beiden Rastpositionen die Schwenkbewegung des ersten Bauteils (2) relativ zu dem zweiten Bauteil (4) in der einen Richtung zu verhindern, wobei das Rastelement (6) und / oder die Rastausnehmung (8) an wenigstens einer Seitenfläche (10, 16) wenigstens eine Stufe (12) aufweisen.

2. Rastgelenk (1) nach Anspruch 1, **dadurch gekennzeichnet, dass** sich das Rast-element (6) zum Eindringen in die Rastausnehmung (8) in radialer Richtung bezüglich der Schwenkachse bewegt.

3. Rastgelenk (1) nach Anspruch 1 oder 2, **dadurch gekennzeichnet, dass** die wenigstens eine Stufe (12) des Rastelement (6) und die wenigstens eine Stufe (12) der Rastausnehmung (8) unterschiedlich breit und / oder unterschiedlich tief ausgebildet sind.

4. Rastgelenk (1) nach einem der vorstehenden Ansprüche, **dadurch gekennzeichnet, dass** das Rastelement (6) und / oder die Rastausnehmung (8) wenigstens eine relativ zur Radialrichtung abgeschrägte Seitenfläche (34, 36) aufweist.

5. Rastgelenk (1) nach Anspruch 4, **dadurch gekennzeichnet, dass** die abgeschrägte Seitenfläche (34) des Rastelementes (6) und die abgeschrägte Seitenfläche (36) der Rastausnehmung (8) den gleichen Winkel relativ zur Radialrichtung aufweisen.

6. Rastgelenk (1) nach einem der vorstehenden Ansprüche, **dadurch gekennzeichnet, dass** zwischen den beiden am weitesten voneinander beabstandeten Rastpositionen ein Schwenkwinkel um die Schwenkachse zwischen 0° und 30°, bevorzugt zwischen 5° und 15°, besonders bevorzugt von 10° liegt.

7. Rastgelenk (1) nach einem der vorstehenden Ansprüche, **dadurch gekennzeichnet, dass** das Rastelement (6) derart angeordnet ist, dass es beim Verschwenken des ersten Bauteils (2) relativ zu dem zweiten Bauteil (4) auf einer Fläche (14) des ersten Bauteils (2) entlanggleitet, in der sich die Rast-ausnehmung (8) befindet.

8. Rastgelenk (1) nach einem der vorstehenden Ansprüche, **gekennzeichnet durch** eine Löseeinrichtung (11), die eingerichtet ist, das Rastelement (6) aus der Rastausnehmung (8) zu entfernen.

9. Orthopädietechnische Einrichtung, mit wenigstens einem Rastgelenk (1) nach einem der vorstehenden Ansprüche.

## Claims

1. A locking hinge (1), comprising
a first component (2) and
a second component (4)
which are mounted on each other so as to be pivotable relative to each other about a pivot axis,
wherein the first component (2) has a locking recess (8) and the second component (4) has a locking element (6) which is designed, in a locking position of the first component (2) relative to the second component (4), to penetrate the locking recess (8) and thus to prevent a pivoting movement of the first component (2) relative to the second component (4) in at least one direction,
**characterized in that** the locking element (6) is designed to penetrate the locking recess (8) to different depths in at least two different locking positions and thus, in the two locking positions, to prevent the pivoting movement of the first component (2) relative to the second component (4) in the one direction, wherein the locking element (6) and/or the locking recess (8) have at least one step (12) on at least one side surface (10, 16).

2. The locking hinge (1) as claimed in claim 1, **characterized in that,** in order to penetrate the locking recess (8), the locking element (6) moves in the radial direction with respect to the pivot axis.

3. The locking hinge (1) as claimed in claim 1 or 2, **characterized in that** the at least one step (12) of the locking element (6) and the at least one step (12) of the locking recess (8) are designed to differ in width and/or to differ in depth.

4. The locking hinge (1) as claimed in one of the preceding claims, **characterized in that** the locking element (6) and/or the locking recess (8) has at least one side surface (34, 36) which is beveled relative to the radial direction.

5. The locking hinge (1) as claimed in claim 4, **characterized in that** the beveled side surface (34) of the locking element (6) and the beveled side surface (36) of the locking recess (8) have the same angle relative to the radial direction.

6. The locking hinge (1) as claimed in one of the preceding claims, **characterized in that** a pivoting angle about the pivot axis of between 0° and 30°, preferably between 5° and 15°, particularly preferably of 10°, lies between the two locking positions having the furthest distance from each other.

7. The locking hinge (1) as claimed in one of the preceding claims, **characterized in that** the locking element (6) is arranged in such a manner that, during the pivoting of the first component (2) relative to the second component (4), said locking element slides along a surface (14) of the first component (2), in which surface the locking recess (8) is located.

8. The locking hinge (1) as claimed in one of the preceding claims, **characterized by** a release device (11) which is designed to remove the locking element (6) from the locking recess (8).

9. An orthopedic device, comprising at least one locking hinge (1) as claimed in one of the preceding claims.

## Revendications

1. Articulation à encliquetage (1) comprenant
un premier composant (2) et
un deuxième composant (4)
qui sont montés l'un à l'autre de manière à pouvoir pivoter l'un par rapport à l'autre autour d'un axe de pivotement,
le premier composant (2) présentant un évidement d'encliquetage (8) et le deuxième composant (4) présentant un élément d'encliquetage (6) conçu pour pénétrer dans l'évidement d'encliquetage (8) et pour ainsi empêcher tout mouvement de pivotement du premier composant (2) par rapport au deuxième composant (4) dans au moins une direction dès que le premier composant (2) atteint une position d'encliquetage par rapport au deuxième composant (4),
**caractérisée en ce que** l'élément d'encliquetage (6) est conçu pour pénétrer dans l'évidement d'encliquetage (8) à différentes profondeurs, dans au moins deux positions d'encliquetage différentes, et pour ainsi empêcher tout mouvement de pivotement du premier composant (2) par rapport au deuxième composant (4) dans ladite direction, dans les deux positions d'encliquetage, l'élément d'encliquetage (6) et/ou l'évidement d'encliquetage (8) présentant au moins un épaulement (12) sur au moins une face latérale (10, 16).

2. Articulation à encliquetage (1) selon la revendication 1,
**caractérisée en ce que**, pour pénétrer dans l'évidement d'encliquetage (8), l'élément d'encliquetage (6) se déplace dans la direction radiale par rapport à l'axe de pivotement.

3. Articulation à encliquetage (1) selon la revendication 1 ou 2,
**caractérisée en ce que** ledit au moins un épaulement (12) de l'élément d'encliquetage (6) et ledit au moins un épaulement (12) de l'évidement d'encliquetage (8) présentent des largeurs et/ou des profondeurs différentes.

4. Articulation à encliquetage (1) selon l'une des revendications précédentes,
**caractérisée en ce que** l'élément d'encliquetage (6) et/ou l'évidement d'encliquetage (8) présente(nt) au moins une surface latérale (34, 36) inclinée par rapport à la direction radiale.

5. Articulation à encliquetage (1) selon la revendication 4,
**caractérisée en ce que** la surface latérale inclinée (34) de l'élément d'encliquetage (6) et la surface latérale inclinée (36) de l'évidement d'encliquetage (8) présentent le même angle par rapport à la direction radiale.

6. Articulation à encliquetage (1) selon l'une des revendications précédentes,
**caractérisée en ce que** l'angle de pivotement autour de l'axe de pivotement entre les deux positions d'encliquetage les plus éloignées est compris entre 0° et 30°, de préférence entre 5° et 15°, et est de manière particulièrement préférée égal à 10°.

7. Articulation à encliquetage (1) selon l'une des revendications précédentes,
**caractérisée en ce que** l'élément d'encliquetage (6) est agencé de telle sorte que, lors du pivotement du premier composant (2) par rapport au deuxième composant (4), il coulisse le long d'une surface (14) du premier composant (2) dans laquelle se trouve l'évidement d'encliquetage (8).

8. Articulation à encliquetage (1) selon l'une des revendications précédentes,
**caractérisée par** un dispositif de libération (11) conçu pour extraire l'élément d'encliquetage (6) hors de l'évidement d'encliquetage (8).

9. Dispositif orthopédique comprenant au moins une articulation à encliquetage (1) selon l'une des revendications précédentes.
